(19) Europäisches Patentamt
European Patent Office
Office européen des brevets

(11) Veröffentlichungsnummer: **0 129 125**
**A1**

(12) # EUROPÄISCHE PATENTANMELDUNG

(21) Anmeldenummer: 84106291.2

(22) Anmeldetag: 01.06.84

(51) Int. Cl.³: C 07 D 207/444

(30) Priorität: 11.06.83 DE 3321157

(43) Veröffentlichungstag der Anmeldung: 27.12.84 Patentblatt 84/52

(84) Benannte Vertragsstaaten: DE FR GB IT NL

(71) Anmelder: BAYER AG
Konzernverwaltung RP Patentabteilung
D-5090 Leverkusen 1 Bayerwerk(DE)

(72) Erfinder: Bauer, Dieter, Dr.
Bodelschwinghstrasse 127
D-4150 Krefeld(DE)

(72) Erfinder: Dohm, Heinz, Dr.
Heyenbaumstrasse 86b
D-4150 Krefeld(DE)

(72) Erfinder: Thiel, Reinhard, Dr.
Buschstrasse 215 a
D-4150 Krefeld 1(DE)

(72) Erfinder: Hübner, Armin, Dr.
Doerperhofstrasse 14
D-4150 Krefeld(DE)

(72) Erfinder: Schneider, Joachim, Dr.
Heyenbaumstrasse 86a
D-4150 Krefeld 1(DE)

(54) **Verfahren zur Herstellung von 1H-Pyrrol-2,5-dionen.**

(57) 1H-Pyrrol-2, 5-dione werden in der Weise hergestellt, daß man in einem 1-stufigen Verfahren primäre Amine mit cyclischen Anhydriden in Gegenwart von inerten organischen, mit Wasser nicht mischbaren Lösungs- und/oder Verdünnungsmitteln sowie in Gegenwart von Säuren bei erhöhter Temperatur umsetzt und das bei der Reaktion entstehende Wasser durch Destillation aus dem Reaktionsgemisch entfernt.

EP 0 129 125 A1

BAYER AKTIENGESELLSCHAFT
Konzernverwaltung RP
Patentabteilung

5090 Leverkusen, Bayerwerk

0129125

Bg/bc/c

# Verfahren zur Herstellung von 1H-Pyrrol-2,5-dionen

Die Erfindung betrifft ein Verfahren zur Herstellung von 1H-Pyrrol-2,5-dionen durch Umsetzung von primären Aminen mit cyclischen Anhydriden in Gegenwart von inerten organischen, mit Wasser nicht mischbaren Lösungs- und/oder Verdünnungsmitteln bei erhöhter Temperatur.

Aus der US-PS 2 262 262 ist es z.B. bekannt, N-Phenylmaleinimid durch Umsetzung von Maleinsäure und Anilin in Gegenwart von Dichlorbenzol durch Zufügen von äquimolaren Mengen an Thionylchlorid herzustellen. N-Phenylmaleinimid wird nach diesem Verfahren in einer Ausbeute von nur 47 % der Theorie erhalten (vgl. Beispiel 1 der US-PS 2 262 262).

Es ist weiterhin bekannt, N-Phenylmaleinimid in einer Zweistufensynthese herzustellen, wobei zunächst das Maleinsäurehalbanilid isoliert und anschließend in Gegenwart von überschüssigem Acetanhydrid und Natriumacetat das Maleinsäurehalbanilid zum entsprechenden Maleinimid cyclisiert wird (vgl. US-PS 2 444 536).

Außerdem lassen sich N-Arylmaleinimide zweistufig so herstellen, daß zunächst das Maleinsäurehalbanilid in einem inerten Lösungsmittel ausgefällt und anschließend unter Zugabe einer Säure als Katalysator zum Maleinimid cyclisiert wird (vgl. GB-Patent 1533068).

Es wurde nun ein Verfahren zur Herstellung von 1H-Pyrrol-2,5-dionen der Formel

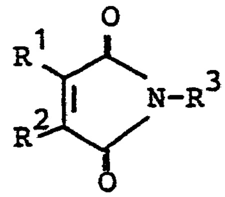

(I) ,

in der

$R^1$ und $R^2$ gleich oder verschieden sind und für Wasserstoff, Halogen, einen gegebenenfalls substituierten niederen Alkyl- oder Arylrest stehen und

$R^3$ einen gegebenenfalls substituierten aliphatischen, cycloaliphatischen oder aromatischen Rest darstellt,

durch Umsetzung eines primären Amins der Formel

$$R^3\text{-}NH_2 \qquad \text{(II)} ,$$

in der

$R^3$ die o.g. Bedeutung hat,

mit einem cyclischen Anhydrid der Formel

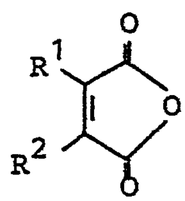

(III) ,

in der

$R^1$ und $R^2$ die oben angegebene Bedeutung haben,

in Gegenwart von inerten organischen, mit Wasser nicht mischbaren Lösungs- und/oder Verdünnungsmitteln bei erhöhter Temperatur gefunden, das dadurch gekennzeichnet ist, daß man die Umsetzung einstufig in Gegenwart von Säuren durchführt und das bei der Reaktion entstehende Wasser durch Destillation aus dem Reaktionsgemisch entfernt.

Als Säuren können solche organischen oder anorganischen Verbindungen in das erfindungsgemäße Verfahren eingesetzt werden, deren $pK_s$-Wert $\leq 4$, vorzugsweise $\leq 2$, insbesondere $\leq 1$, beträgt. Solche Verbindungen sind beispielsweise anorganische und organische Säuren. Als anorganische Säuren, die in das erfindungsgemäße Verfahren eingesetzt werden können, seien genannt:Phosphorige Säure, Phosphorsäure, Pyrophosphorsäure, Polyphosphorsäure, Schwefelsäure, schweflige Säure, Bromwasserstoffsäure, Jodwasserstoffsäure, Fluorsulfonsäure und/oder saure Ionenaustauscher.

Als organische Säuren können eingesetzt werden: Chloressigsäure, Dichloressigsäure, Trichloressigsäure, Fluoressigsäure, Difluoressigsäure, Trifluoressigsäure, Bromessigsäure, Oxalsäure und/oder Sulfonsäuren, wie Methansulfonsäure, Ethylsulfonsäure, Octylsulfonsäure, Decansulfonsäure, Dodecansulfonsäure, Tetradecansulfonsäure, Hexadecansulfonsäure, Perfluormethylsulfonsäure, Perfluorbutansulfonsäure, Perfluoroctansulfonsäure, Benzolsulfonsäure, Chlorbenzolsulfonsäure, Methylbenzolsulfonsäure und Dodecylbenzolsulfonsäure.

Vorzugsweise werden in das erfindungsgemäße Verfahren solche organischen und/oder anorganischen Säuren eingesetzt, die leicht zugänglich und zudem preiswert sind.

In diesem Zusammenhang seien besonders die Phosphor- und die Schwefelsäure genannt. Dabei wird die Phosphorsäure üblicherweise als etwa 10 bis 100 gew.-%ige Phosphorsäure, bevorzugt als 60 bis 90 gew.-%ige Phosphorsäure, besonders bevorzugt als 85 bis 89 gew.-%ige Phosphorsäure, in das erfindungsgemäße Verfahren eingesetzt. Als Schwefelsäure verwendet man üblicherweise eine etwa 10 bis 100, bevorzugt 80 - 100% gew.-%ige Schwefelsäure oder eine konzentrierte Schwefelsäure mit einem Gehalt von etwa 60 bis 100 Gew.-% $SO_3$, vorzugsweise 70 bis 90 Gew.-% $SO_3$.

Die Menge der einzusetzenden Säuren beträgt im allgemeinen etwa 0,01 bis 20 Gew.-%, bezogen auf das eingesetzte cyclische Anhydrid, vorzugsweise 0,1 bis 10 Gew.-%, besonders bevorzugt 0,3 bis 7 Gew.-%. Die optimale Menge der einzusetzenden Säuren, die u.a. von den jeweils eingesetzten Reaktanden abhängt, kann durch Vorversuche leicht ermittelt werden. Das gegebenenfalls in den Säuren noch vorhandene Wasser ist für die erfindungsgemäße Umsetzung nicht kritisch, da es während der Reaktion zusammen mit dem Reaktionswasser aus dem Reaktionsgemisch durch Destillation ausgetragen wird.

Als primäre aliphatische Amine können solche der allgemeinen Formel (II)

$$R^3\text{-}NH_2 \qquad (II),$$

in der

$R^3$ einen gegebenenfalls substituierten aliphatischen, cycloaliphatischen oder aromatischen Rest darstellt,

in das erfindungsgemäße Verfahren eingesetzt werden.

Als aliphatische Reste kommen solche mit 1 bis 30 C-Atomen, bevorzugt 1 bis 20 C-Atomen, in Frage, wie der Methyl-, Ethyl-, Propyl-, Isopropyl-, Butyl-, Isobutyl-, Hexyl-, Octyl-, 2-Ethylhexyl-, Decyl-, Dodecyl-, Tetradecyl-, Hexadecyl-, Octadecyl-, Eicosyl- oder der Oleylrest.

Als cycloaliphatische Reste kommen solche mit 4 bis 12 C-Atomen, bevorzugt 5 bis 10 C-Atomen, in Frage, wie der Cyclopentyl- oder der Cyclohexylrest, bevorzugt der Cyclohexylrest.

Als aromatische Reste kommen solche mit 6 bis 12 C-Atomen, bevorzugt 6 bis 10 C-Atomen, in Frage, wie der Phenyl-, Xylyl-, Tolyl- oder Naphthylrest.

Als Substituenten der Amine kommen solche in Betracht, die unter den Bedingungen des erfindungsgemäßen Verfahrens nicht in unerwünschter Weise reagieren, sich also inert verhalten. Als Substituenten der aliphatischen-, cycloaliphatischen- und der aromatischen Amine werden z.B. genannt: Halogene, wie Fluor, Brom, Chlor oder Jod, niedere Alkyl-, Alkoxy- und Alkylmercaptoreste

mit 1 bis 4 Kohlenstoffatomen, wie der Methyl-, Ethyl-, Propyl-, Isopropyl-, Butyl-, sec.-Butyl-, tert.-Butyl-, Methoxy-, Ethoxy-, Propoxy-, Butoxy-, Methylmarcapto-, Ethylmercapto-, Propylmercapto- oder der Butylmercaptorest, sowie Arylreste, wie Phenyl.

Die primären Amine der Formel (II) können dabei ein- oder mehrfach durch die genannten Substituenten substituiert sein.

Besonders bevorzugt werden in das erfindungsgemäße Verfahren cycloaliphatische Amine der Formel (IV)

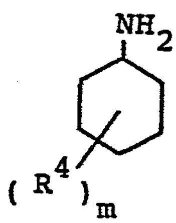

(IV)

und aromatische Amine der Formel (V)

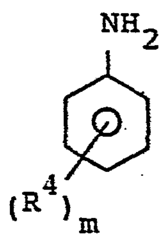

(V) ,

eingesetzt in denen

$R^4$ für Halogen, einen niederen Alkyl-, Alkoxy-, Alkylmercaptorest mit 1 bis 4 Kohlenstoffatomen oder für einen Arylrest steht,

m eine der Zahlen 0, 1, 2, 3, 4, 5 bedeutet, wobei im Falle für m = 2, 3, 4 oder 5 die Reste $R^4$ verschieden sein können.

Ganz besonders bevorzugt werden solche cycloaliphatischen oder aromatischen Amine in das erfindungsgemäße Verfahren

eingesetzt, in denen $R^4$ für Wasserstoff, Chlor, Methyl oder Ethyl steht und m die Zahlen 0, 1 oder 2 bedeutet.

Als beispielhafte Vertreter für die aliphatischen, cycloaliphatischen oder aromatischen Amine, die in das erfindungsgemäße Verfahren eingesetzt werden können, seien genannt: Butylamin, Isobutylamin, Hexylamin, Octylamin, 2-Ethylhexylamin, Decylamin, Dodecylamin, Tetradecylamin, Hexadecylamin, Octadecylamin, Eicosylamin, Oleylamin, Cyclohexylamin, Methylcyclohexylamin, Chlorcyclohexylamin, Anilin, o,m,p-Chloraniline, o,m,p-Bromaniline, o,m,p-Toluidine, Xylidine, o,m,p-Ethylaniline, o,m,p-Anisidine, o,m,p-Thiomethylaniline oder Phenylaniline. Besonders bevorzugt werden eingesetzt Butylamin, Cyclohexylamin, Ethylhexylamin, Anilin, o,m,p-Chloranilin, und o,m,p-Toluidin.

Als cyclische Anhydride, die in das erfindungsgemäße Verfahren eingesetzt werden können, kommen solche der allgemeinen Formel (III)

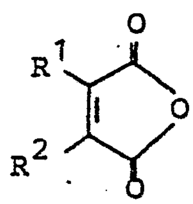

(III) ,

wobei

$R^1$ und $R^2$ gleich oder verschieden sind und für Wasserstoff, Halogen, einen gegebenenfalls substituierten niederen Alkyl- oder Arylrest stehen,

in Frage.

Als Halogene seien genannt: Fluor, Brom, Chlor oder Jod, bevorzugt Brom und Chlor;
als niedere Alkylreste solche mit 1 bis 4, bevorzugt 1 bis 3, Kohlenstoffatomen, wie Methyl, Ethyl, n-Propyl, iso-Propyl, n-Butyl, iso-Butyl, bevorzugt Methyl und Ethyl;
als Arylreste solche mit 6 bis 12 Kohlenstoffatomen, bevorzugt 6 bis 10 Kohlenstoffatomen, wie Phenyl, Tolyl, Xylyl und Naphthyl, bevorzugt Phenyl und Tolyl.

Die Alkyl- oder die Arylreste können substituiert sein durch Halogen und/oder Alkoxy, bevorzugt durch Brom, Chlor und Methoxy.

Nach dem erfindungsgemäßen Verfahren werden die primären Amine und die cyclischen Anhydride im allgemeinen in äquimolaren Mengen zur Reaktion gebracht. Es ist jedoch auch möglich, einen Überschuß an cyclischen Anhydriden einzusetzen. Dabei kann das überschüssige, nicht-umgesetzte cyclische Anhydrid gegebenenfalls nach der Reaktion wiedergewonnen und erneut in die Reaktion eingesetzt werden. Bevorzugt werden die primären Amine und die cyclischen Anhydride in einem molaren Verhältnis von 0,5 bis 2:1, bevorzugt 0,8 bis 1,05:1 (prim. Amin: cyclisches Anhydrid), in das erfindungsgemäße Verfahren eingesetzt.

Die Reaktionstemperatur des erfindungsgemäßen Verfahrens liegt üblicherweise im Bereich von etwa 75 bis 300°C, vorzugsweise bei 80 bis 250°C, insbesondere bei 100 bis 200°C.

Als organische Lösungs- und/oder Verdünnungsmittel können solche in das erfindungsgemäße Verfahren eingesetzt werden, die mit Wasser nicht mischbar sind und sich unter den Reaktionsbedingungen inert verhalten. Bevorzugt sind solche Lösungs- und/oder Verdünnungsmittel, die einen Siedepunkt von etwa 75 bis 300°C, vorzugsweise 80 bis 250°C, besonders bevorzugt 100 bis 200°C, besitzen. Die Lösungs- und/oder Verdünnungsmittel können sowohl einzeln als auch im Gemisch untereinander eingesetzt werden.

Im allgemeinen werden die Lösungs- und/oder Verdünnungsmittel in einer Menge eingesetzt, die mindestens etwa 25 Gew.-% des Gesamtgewichtes der Reaktionspartner beträgt, als bevorzugt wird eine Menge eingesetzt, die 30 Gew.-%, bezogen auf das Gesamtgewicht der Reaktionspartner, nicht unterschreitet. Die optimale Menge an Lösungs- und/oder Verdünnungsmittel kann leicht durch Vorversuche ermittelt werden.

Als organische Lösungs- und/oder Verdünnungsmittel seien genannt: Aliphatische oder aromatische Kohlenwasserstoffe, wie n-Heptan, Isooctan, Methylcyclohexan, 2,5-Dimethylhexan, n-Octan, n-Nonan, Dekan, trans/cis-Dekalin, Toluol, Ethylbenzol, o-, m-, p-Xylol, Cumol, n-Propylbenzol, Mesitylen, p-Cymol, n-Butylbenzol und Durol, bevorzugt n-Heptan, Isooctan, Toluol, o-, m-, p-Xylol, Durol und Ethylbenzol, und/oder deren Chlorierungsprodukte wie n-Butylchlorid, n-Amylchlorid, n-Hexyl-, Cyclohexyl-, n-Heptyl-, n-Octylchlorid-, Dichlorethan, Tetrachlorethylen, Chlorbenzol, Brombenzol, Chlor-

toluol, Bromtoluol, Dichlorbenzol, bevorzugt Dichlorethan, Chlorbenzol und Dichlorbenzol; Ester, wie Buttersäureethylester, Buttersäuremethylester, Essigsäurebutylester, -amylester, n-Hexylester, Diethylcarbonat, bevorzugt Buttersäureethylester, Essigsäurebutylester und Diethylcarbonat; Ether, wie Diisopropylether, Dipropylether, Dibutylether, Anisol, Phenetol, bevorzugt Dibutylether und Anisol.

Das erfindungsgemäße Verfahren kann am Beispiel der Umsetzung von Anilin mit Maleinsäureanhydrid durch folgende Gleichung erläutert werden:

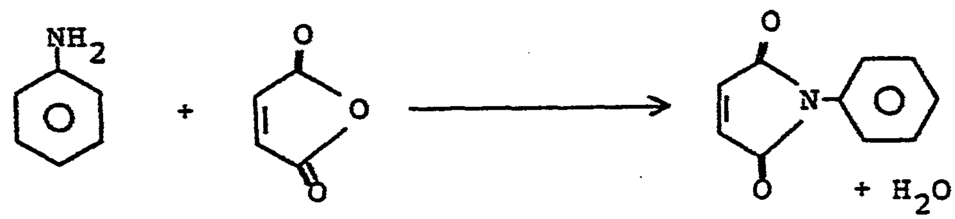

Üblicherweise wird das erfindungsgemäße Verfahren in der Weise durchgeführt, daß die Säuren bei Raumtemperatur oder erhöhter Temperatur dem inerten organischen Lösungs- und/oder Verdünnungsmittel kontinuierlich oder in kleinen Portionen über einen längeren Zeitraum zugeführt werden, anschließend diesem Gemisch das cyclische Anhydrid zugegeben und bei Siedetemperatur des Lösungsmittels das primäre Amin zugetropft wird. Die Umsetzung erfolgt im allgemeinen bei der Siedetemperatur des inerten organischen Lösungs- und/oder Verdünnungsmittels, wobei das sich

bildende Reaktionswasser durch eine geeignete Vorrichtung destillativ, kontinuierlich aus dem Reaktionsgemisch abgetrennt und das Lösungsmittel wieder dem Reaktionsgemisch zugeführt wird. Die Reaktion ist dann beendet, wenn sich die theoretisch zu erwartende Menge an Reaktionswasser gebildet hat. Die Reaktionszeit hängt von verschiedenen Parametern ab, wie der Menge und der Art des Katalysators, der Menge und der Art des eingesetzten organischen Lösungs- und/oder Verdünnungsmittels und der Reaktivität der Reaktionspartner.

Das erfindungsgemäße Verfahren weist gegenüber dem Stand der Technik den Vorteil auf, daß in einem einstufigen Verfahren ohne Bildung schwer handhabbarer Suspensionen, die eine große Menge inerten Lösungsmittels erfordern um sie rührbar zu halten, 1 H-Pyrrol-2,5-dione in besonders guten Ausbeuten erhalten werden. Außer Reaktionsvolumen wird Energie für das Abdestillieren des großen Lösungsmittelanteils eingespart. Zudem werden für das erfindungsgemäße Verfahren erheblich kürzere Reaktionszeiten benötigt.

Die guten Ausbeuten, die bei dem erfindungsgemäßen Verfahren erzielt werden, sind besonders überraschend, da bekanntlicherweise 1 H-Pyrrol-dione unter sauren Reaktionsbedingungen in Lösung leicht zur Zersetzung und zur Polymerisation neigen. Aus diesem Grunde wurden bislang in einem einstufigen Verfahren unter sauren Bedingungen nur unbefriedigende Ausbeuten erhalten (vgl. US-PS 22 62 262, Beispiel 1).

Die durch das erfindungsgemäße Verfahren herstellbaren 1H-Pyrrol-2,5-dione sind z. B. wertvolle Comonomere bei der Polymerisation von Alkenen (vgl. z. B. DE-OS 31 48 965 und DE-OS 27 12 171).

Die nachfolgenden Beispiele sollen das erfindungsgemäße Verfahren verdeutlichen, ohne es jedoch auf diese Beispiele einzuschränken.

Beispiele

Nach der folgenden allgemeinen Methode wurden die in der Tabelle 1 angegebenen Beispiele 1 bis 14 durchgeführt:

In einem mit Rührer, Kolonne, Rückflußkühler und Wasserabscheider versehenen Rundkolben wurden Lösungsmittel, Katalysator und 98 g (1 Mol) Maleinsäureanhydrid (MSA) eingebracht. Dann ließ man bei Rückflußbedingungen unter Wasserabtrennung 1 Mol Anilin (93 g) zutropfen. Nach einer Nachreaktionszeit von 30 Minuten wurde durch Destillation bei 12 Torr aufgearbeitet und die gelbe Fraktion vom $Kp_{12}$ = 166-168°C getrennt aufgefangen. Die gaschromatographische Analyse ergab N-Phenylmaleinimid mit einem Gehalt von 99,0 bis 99,7 %.

Bei den aufgeführten Ausbeuten handelt es sich um Angaben in % der theoretisch möglichen Ausbeuten, bezogen auf Maleinsäureanhydrid.

Tabelle 1

N-Phenylmaleinimid

| Beispiel | Katalysator (bezogen auf MSA + Anilin) | Lösungsmittel | Reaktionszeit (h) | Ausbeute (%) |
|---|---|---|---|---|
| 1 | 0,5 % $H_2SO_4$ (96 %ig) | o-Xylol 150 ml | 1-2 | 88 |
| 2 | 2,5 % $H_3PO_3$ | " " | 1-2 | 92 |
| 3 | 1,5 % $H_3PO_4$ (70 %ig) | " " | 1-2 | 90 |
| 4 | 1,5 % Alkylsulfonsäure | " " | 1-2 | 90 |
| 5 | 1,5 % Benzolsulfonsäure | " " | 1-2 | 89 |
| 6 | 1 % Methansulfonsäure | " " | 1-2 | 88 |
| 7 | 1 % $H_2SO_4$ (96 %ig) | " " | 1 | 87 |
| 8 | 1 % $H_2SO_4$ (96 %ig) | " 500 ml | 1-2 | 90 |
| 9 | 0,5 % $H_2SO_4$ (96 %ig) | o,m,p-Xylol-Gemisch 150 ml | 1-2 | 89 |
| 10 | 1 % $H_2SO_4$ (96 %ig) | Toluol/o-Xylol 100 ml/50 ml | 2-3 | 93 |
| 11 | 1 % $H_2SO_4$ (96 %ig) | Diethylcarbonat 150 ml | 1-2 | 90 |
| 12 | 1 % $H_2SO_4$ (96 %ig) | Toluol 150 ml | 2-3 | 87 |
| 13 | 1 % $H_2SO_4$ (96 %ig) | Ethylbenzol 150 ml | 1-2 | 92 |
| 14 | 1 % $H_2SO_4$ (96 %ig) | Mesitylen 150 ml | 1 | 93 |

Beispiel 15

Zur Lösung von 98 g MSA (1 Mol) in 300 ml o-Dichlorbenzol, die sich in einem wie unter Beispiel 1 beschriebenen Reaktionsgefäß befand, wurden 2 g 85 %ige Phosphorsäure gegeben und innerhalb von 1 Stunde bei 155 °C 93 g (1 Mol) Anilin unter Rühren zugetropft. Nach 1 Stunde Nachreaktionszeit war die theoretische Menge Wasser abdestilliert. Eindampfen der Lösung unter vermindertem Druck, Extraktion der Phosphorsäure mit Wasser und Destillation lieferten das N-Phenylmaleinimid in 94 %iger Ausbeute; Schmp. 89 °C.

Beispiel 16

Wie in Beispiel 15 beschrieben, wurden 98 g MSA (1 Mol) und 99 g (1 Mol) Cyclohexylamin zur Reaktion gebracht. Ausbeute: 80 % der Theorie an N-Cyclohexylmaleinimid; Schmp. 87°C; Lit. 88-89°C.

Beispiel 17

Wie in Beispiel 15 beschrieben, wurden 98 g MSA (1 Mol) und ~~127,5 g~~ (1 Mol) p-Cl-anilin umgesetzt. Ausbeute: 85 % der Theorie an N-(p-Chlor-phenyl)-maleinimid; Schmp. 109-110°C; Lit. 110°C.

Beispiel 18

Wie in Beispiel 15 beschrieben, ließ man 98 g MSA (1 Mol) mit 107 g p-Toluidin reagieren.

Ausbeute: 86 % der Theorie an N-(p-Tolyl)-maleinimid; Schmp. 146-147°C; Lit. 150°C.

Beispiel 19

Wie in Beispiel 15 beschrieben, ließ man 98 g MSA (1 Mol) und 73 g n-Butylamin reagieren.
Ausbeute: 80 % der Theorie an N-(n-Butyl)-maleinimid; $Kp_9$: 92°C; $n_D^{20}$: 1,4789.

Beispiel 20

In einem 250 ml-Rundkolben mit Wasserabscheider wurde eine Lösung von 31,2 g (0,36 Mol) Citraconsäureanhydrid in 150 ml Mesitylen vorgelegt und dazu innerhalb von 1 Stunde 26 g Anilin bei 40°C zutropfen gelassen. Nach Zugabe von 0,5 g $H_3PO_4$ (85 %ig) ließ man 1 Stunde bei Rückflußtemperatur unter Wasserabtrennung reagieren und engte die Lösung unter vermindertem Druck ein. Destillation des Rückstandes ergab 49 g ≙ 73 % der Theorie an N-Phenylcitraconimid.
Schmp. 97°C; Lit. 98°C.

Beispiel 21

Wie unter Beispiel 20 beschrieben, wurden 30,5 g (0,24 Mol) Dimethylmaleinsäureanhydrid mit 22,5 g Anilin zur Reaktion gebracht.
Ausbeute: 37 g ≙ 75 % der Theorie an N-Phenyldimethylmaleinimid; Schmp. 92°C; Lit. 96°C.

Patentansprüche

1) Verfahren zur Herstellung von 1H-Pyrrol-2,5-dionen der Formel

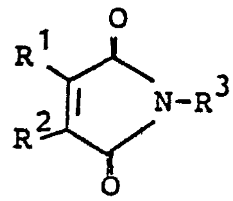

,

in der

$R^1$ und $R^2$ gleich oder verschieden sind und für Wasserstoff, Halogen, einen gegebenenfalls substituierten niederen Alkyl- oder Arylrest stehen und

$R^3$ einen gegebenenfalls substituierten aliphatischen, cycloaliphatischen oder aromatischen Rest darstellt,

durch Umsetzung eines primären Amins der Formel

$$R^3\text{-}NH_2 \quad ,$$

in der

$R^3$ die oben angegebene Bedeutung hat,

mit einem cyclischen Anhydrid der Formel

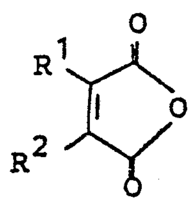

,

0129125

in der
$R^1$ und $R^2$ die oben angegebene Bedeutung haben,

in Gegenwart von inerten organischen, mit Wasser nicht mischbaren Lösungs- und/oder Verdünnungsmitteln bei erhöhter Temperatur, dadurch gekennzeichnet, daß man die Umsetzung einstufig in Gegenwart von Säuren durchführt und das bei der Reaktion entstehende Wasser durch Destillation aus dem Reaktionsgemisch entfernt.

2) Verfahren nach Anspruch 1, dadurch gekennzeichnet, daß man organische und/oder anorganische Säuren einsetzt, deren $pK_s$-Wert $\leq$ 4 beträgt.

3) Verfahren nach Ansprüchen 1 und 2, dadurch gekennzeichnet, daß man organische und/oder anorganische Säuren einsetzt, deren $pK_s$-Wert $\leq$ 2 beträgt.

4) Verfahren nach Ansprüchen 1 bis 3, dadurch gekennzeichnet, daß man als Säuren Phosphor- und/oder Schwefelsäure einsetzt.

5) Verfahren nach Anspruch 4, dadurch gekennzeichnet, daß man eine 10 bis 100 gew.-%ige Phosphorsäure einsetzt.

6) Verfahren nach Anspruch 4, dadurch gekennzeichnet, daß man eine 10 bis 100 gew.-%ige Schwefelsäure einsetzt.

7) Verfahren nach Anspruch 4, dadurch gekennzeichnet, daß man eine konzentrierte Schwefelsäure mit einem Gehalt von 60 bis 100 Gew.-% $SO_3$ einsetzt.

8) Verfahren nach Ansprüchen 1 bis 7, dadurch gekennzeichnet, daß man die Säuren in einer Menge von 0,01 bis 20 Gew.-%, bezogen auf das eingesetzte cyclische Anhydrid, einsetzt.

9) Verfahren nach Ansprüchen 1 bis 8, dadurch gekennzeichnet, daß man die Säuren in einer Menge von 0,1 bis 10 Gew.-%, bezogen auf das eingesetzte cyclische Anhydrid, einsetzt.

10) Verfahren nach Ansprüchen 1 bis 9, dadurch gekennzeichnet, daß man die Säuren in einer Menge von 0,3 bis 7 Gew.-%, bezogen auf das eingesetzte cyclische Anhydrid, einsetzt.

Europäisches Patentamt

# EUROPÄISCHER RECHERCHENBERICHT

0129125
Nummer der Anmeldung

EP 84 10 6291

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 3) |
|---|---|---|---|
| X | DE-A-2 649 743 (IHARA CHEMICAL CO. LTD.)<br>* Anspruch 7 *<br>--- | 1-10 | C 07 D 207/444 |
| X | GB-A-2 043 054 (ABOTT LAB.)<br>* Insgesamt *<br>--- | 1-10 | |
| X | FR-A-2 444 667 (RHONE-POULENC)<br>* Ansprüche *<br>--- | 1-10 | |
| X | DE-A-2 626 795 (CIBA)<br>* Beispiel 1 *<br>--- | 1-10 | |
| A | FR-A-1 508 526 (KOPPERS CO. INC.)<br>* Insgesamt *<br>--- | 1 | RECHERCHIERTE SACHGEBIETE (Int. Cl. 3) |
| D,A | US-A-2 262 262 (J.H. SPEER)<br>--- | | C 07 D 207/00 |
| D,A | US-A-2 444 536 (N.E. SEARLE)<br>----- | | |

Der vorliegende Recherchenbericht wurde für alle Patentansprüche erstellt.

| Recherchenort | Abschlußdatum der Recherche | Prüfer |
|---|---|---|
| DEN HAAG | 24-09-1984 | MAISONNEUVE J.A. |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503. 03.82